# DEMANDE DE BREVET EUROPEEN

(11) **EP 0 800 820 A2**
(43) Date de publication de la demande: **15.10.1997**
(21) Numéro de dépôt: 97400674.4
(22) Date de dépôt: 25.03.1997
(51) Int. Cl.: A61K 7/48

(54) **Composition cosmétique comprenant des corps gras Pâteux et des composés volatils**

(30) Priorité: 19.04.1996 FR 9605023; 05.07.1996 FR 9608420
(71) Demandeur: L'OREAL, 75008 Paris (FR)
(72) Inventeur: Agostini, Isabelle, 92290 Chatenay Malabry (FR)
(74) Mandataire: Miszputen, Laurent

(57) **Abrégé**

La présente demande concerne une composition notamment cosmétique pouvant se présenter sous forme d'un stick ou d'une pâte souple, et susceptible d'être utilisée pour le soin et/ou le maquillage de la peau, des semi-muqueuses et/ou des muqueuses, et en particulier des lèvres du visage.
Cette composition comprend notamment des composés volatils et des composés gras pâteux.

## Description

La présente invention a pour objet une composition notamment cosmétique pouvant se présenter sous forme d'un stick ou d'une pâte souple, et susceptible d'être utilisée pour le soin et/ou le maquillage de la peau, des semi-muqueuses et/ou des muqueuses, et en particulier des lèvres du visage.

Les compositions à appliquer sur les lèvres se présentent généralement sous forme de sticks ou bâtons comprenant des corps gras tels que des huiles et des cires, et une phase particulaire généralement composée de charges et de pigments. On a constaté que ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, un vêtement ou la peau. En se déposant, ladite composition laisse une trace sur ledit support. Il s'en suit donc une persistance médiocre de la composition sur la peau ou les muqueuses, d'où la nécessité de renouveler régulièrement son application.

Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. Tous ces phénomènes engendrent un effet inesthétique, que la consommatrice souhaite bien évidemment éviter.

La présente invention a pour but de proposer une composition qui permet d'obtenir un film de très bonne tenue, qui ne transfère pas et ne tache pas un support avec lequel il serait en contact, et qui ne migre pas au cours du temps.

L'invention a donc pour objet une composition comprenant, dans une phase grasse, au moins un composé volatil, moins de 20% en poids d'huile non volatile hydrocarbonée, et 1-40% en poids d'au moins un composé gras pâteux présentant une viscosité à 40°C de 0,1 à 40 Pa.s et/ou un point de fusion de 25-70°C.
Un autre objet de l'invention est une composition sans transfert de maquillage de la peau et/ou des lèvres, comprenant 1-40% en poids d'au moins un composé gras pâteux présentant une viscosité à 40°C de 0,1 à 40 Pa.s et/ou un point de fusion de 25-70°C, au moins un composé volatil et moins de 20% en poids d'huile non volatile hydrocarbonée.
L'invention a également pour objet l'utilisation de l'association d'au moins un composé volatil et d'au moins un composé gras pâteux présentant une viscosité à 40°C de 0,1 à 40 Pa.s et/ou un point de fusion de 25-70°C, afin de diminuer le transfert et/ou la migration, et/ou afin d'améliorer la tenue d'une composition les comprenant.

On a de plus constaté que la composition selon l'invention permet l'obtention d'un film de coloration homogène dû à un bon mouillage des pigments dans les corps gras pâteux; le film est facilement applicable et s'étale facilement sur le support. Le film obtenu présente également une texture légère et est très confortable à porter tout au long de la journée.

La composition selon l'invention comprend donc au moins un composé volatil, qui peut être choisi en particulier parmi les huiles hydrocarbonées ou les huiles siliconées, cycliques ou linéaires, seules ou en mélange. Par composé volatil, on entend dans la présente description, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.
Parmi les huiles siliconées volatiles, on peut citer la cyclotétradiméthylsiloxane, la cyclopentadiméthylsiloxane, la cyclohexadiméthylsiloxane et le méthylhexyldiméthylsiloxane. Parmi les huiles hydrocarbonées volatiles, on peut citer les isoparaffines.
La composition selon l'invention peut comprendre 8-70% en poids, de préférence 15-55%, de composés volatils par rapport au poids total de la composition.

La composition selon l'invention comprend également au moins un composé gras pâteux.
Ce composé est de préférence hydrocarboné, peut être un polymère, et peut également être siliconé et/ou fluoré; il peut aussi se présenter sous forme d'un mélange de différents composés hydrocarbonés et/ou siliconés et/ou fluorés. Dans le cas d'un mélange, on utilise de préférence les composés pâteux hydrocarbonés en proportion majoritaire. On peut définir les composés gras pâteux selon l'invention à l'aide d'au moins une des propriétés physico-chimiques suivantes :
. une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
. un point de fusion de 25-70°C, de préférence 25-55°C.
L'homme du métier peut choisir le mobile permettant de mesurer la viscosité, parmi les mobiles MS-r3 et MS-r4, sur base de ses connaissances générales, de manière à pouvoir réaliser la mesure du composé pâteux testé.

Parmi les composés pâteux susceptibles d'être utilisés dans le cadre de la présente invention, on peut citer les lanolines ou les dérivés de lanoline ayant une viscosité de 18-21 Pa.s, de préférence 19-20,5 Pa.s, et/ou un point de fusion de 30-60°C.
On peut également citer les esters gras, notamment ceux ayant 20 à 45 atomes de carbone (point de fusion de l'ordre de 25-70°C) ainsi que les triglycérides tels que les huiles végétales hydrogénées. Parmi les esters, on peut citer le propionate d'arachidyle, le polylaurate de vinyle et les esters de cholestérol.
On peut aussi citer les corps gras pâteux siliconés tels que les alkyls diméthicones, qui ont un point de fusion de 25-60°C, notamment ceux vendus par Dow Corning sous les noms commerciaux de DC2503 et DC25514.

On peut encore utiliser toute huile usuelle épaissie à l'aide d'un agent épaississant usuel.
Les huiles susceptibles d'être épaissies peuvent être d'origine minérale, végétale, animale et/ou synthétique telle que siliconée, et éventuellement phénylée.
L'agent épaississant peut être choisi parmi les argiles telles que les bentonites ou les hectorites, éventuellement modifiées notamment par du chlorure de distéaryl diméthyl ammonium, ou par du chlorure de stéaryl diméthyl benzyl ammonium, ou par des silicates d'aluminium ou de magnésium, ou encore par des polymères usuels connus pour être susceptibles d'épaissir des huiles.
On peut également utiliser les dérivés d'huile de ricin hydrogénée, tels que le 'THIXINR' de Rheox.

Le ou les composés pâteux peuvent être présents à raison de 1 à 40% en poids, de préférence à raison de 8-35% en poids et encore plus préférentiellement à raison de 15-30% en poids, dans la composition.

La phase grasse peut comprendre, en plus des composés cités ci-dessus, les corps gras usuellement utilisés dans le domaine d'application envisagé, tels que des cires d'origine végétale, minérale, animale et/ou synthétique, notamment siliconée.
Dans un mode de réalisation particulier de l'invention, la composition peut comprendre uniquement des corps gras hydrocarbonés, et en particulier des corps gras pâteux de type lanoline, des composés volatils de type isoparaffines et des cires hydrocarbonées. En effet, on a constaté que le mouillage des pigments était meilleur lorsque les corps gras étaient hydrocarbonés, ce qui conduit à un maquillage de la peau ayant une couleur plus homogène. D'autre part, les corps gras siliconés ne sont pas tous compatibles avec tous les corps gras hydrocarbonés, d'où une plus grande limitation dans les matières premières, au niveau formulation.

On peut employer toute cire connue dans l'art antérieur parmi lesquelles, seules ou en mélange, on peut citer les cires animales, végétales, minérales et synthétiques telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; les cires de silicone.
De préférence, la composition comprend 10-30% en poids de cire, notamment 15-20% en poids, par rapport au poids de la composition.

La composition selon l'invention peut en outre comprendre d'autres corps gras, parmi lesquels on peut citer les polydiméthylsiloxanes (PDMS) et les alkyldiméthicones, ainsi que les silicones modifiées par des groupements aliphatiques et/ou aromatiques, éventuellement fluorés, ou par des groupements fonctionnels tels que des groupements hydroxyles, thiols et/ou amines.
On peut encore citer les huiles notamment hydrocarbonées telles que l'huile de paraffine, de vaseline, le perhydrosqualène, l'huile d'arachide, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides.
On peut encore citer les huiles siliconées et notamment phénylées, telles qu'un polyphénylméthylsiloxane ou un phényltriméthicone, ou un mélange de différentes huiles siliconées phénylées. Ces huiles peuvent notamment répondre à la formule suivante : 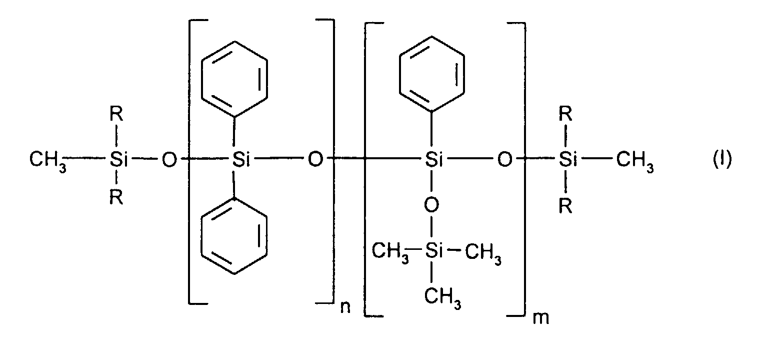 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

Ces corps gras peuvent en particulier être choisis de manière variée par l'homme du métier afin de préparer une composition ayant les propriétés souhaitées, par exemple en consistance, en texture ou en transfert.
En particulier, la composition selon l'invention peut comprendre au moins une cire, de manière à assurer la résistance mécanique, lorsque la composition se présente sous la forme d'un stick. De préférence, les cires entrant ainsi dans la composition peuvent présenter un point de fusion supérieur à 45°C environ, et en particulier supérieur à 55°C, et/ou un indice de pénétration de l'aiguille à 25°C de préférence compris entre 3 et 40.
Lorsqu'elle se présente sous la forme d'une pâte souple ou d'un produit coulé, la composition selon l'invention comprend une quantité moins importante de cire, par exemple de l'ordre de 2-15% en poids.

D'une manière générale, on préfère utiliser les huiles, hydrocarbonées et/ou siliconées, en faible quantité, notamment inférieure à 5% en poids par rapport au poids total de la composition.

Toutefois, on a constaté que l'amélioration de la tenue de la composition selon l'invention, ainsi que son absence de migration et/ou de transfert, pouvait être particulièrement intéressante lorsque la composition comprenait moins de 20% en poids d'huile non volatile hydrocarbonée, de préférence moins de 15% en poids, plus préférentiellement moins de 5% en poids, voire pas d'huiles hydrocarbonées non volatiles du tout, qui peuvent ainsi être remplacées par une quantité plus importante de composés gras pâteux.

La composition peut comprendre également une phase particulaire, généralement présente à raison de 0-30% en poids, de préférence 0-20% en poids, et qui peut comprendre des pigments et/ou des nacres et/ou des charges habituellement utilisés dans les compositions cosmétiques.
Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.
Par nacres, il faut comprendre des particules irisées qui réfléchissent la lumière.
Les pigments peuvent être présents dans la composition à raison de 0-15% en poids de la composition finale, et de préférence à raison de 0-10%. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Les nacres peuvent être présentes dans la composition à raison de 0-20% en poids, de préférence à un taux élevé de l'ordre de 4-15% en poids. Parmi les nacres envisageables, on peut citer le mica recouvert d'oxyde de titane, d'oxyde de fer, de pigment naturel ou d'oxychlorure de bismuth ainsi que le mica titane coloré.
Les charges, qui peuvent être présentes à raison de 0-15% en poids, de préférence 3-10%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le micatitane, la nacre naturelle, le nitrure de bore, les microsphères telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique, tel que des antioxydants, des parfums, des huiles essentielles, des conservateurs, des actifs cosmétiques, des vitamines, des acides gras essentiels, des sphingolipides, des composés auto-bronzants tels que la DHA, des filtres solaires, des tensioactifs, des polymères liposolubles notamment hydrocarbonés, tels que le polybutène, les polyalkylènes, les polyacrylates et les polymères ou dérivés siliconés compatibles avec les corps gras. Ces additifs peuvent être présents dans la composition à raison de 0-10% en poids.
Bien entendu l'homme du métier veillera à choisir les éventuels composés complémentaires, et/ou leur quantité, de telle manière que les propriétés avantageuses de la composition selon l'invention ne soient pas, ou substantiellement pas, altérées par l'adjonction envisagée.

Les procédés de fabrication des compositions selon l'invention ne différent en rien des procédés classiquement utilisés en cosmétique et parfaitement connus de l'homme de l'art. Ces procédés consistent dans le mélange des différents constituants de la composition, préalablement chauffés, puis à leur coulage selon la forme souhaitée.

La composition selon l'invention peut se présenter sous la forme d'un stick ou bâton, sous la forme d'un liquide huileux, éventuellement gélifié, ou encore sous la forme d'une pâte souple dont on peut mesurer la viscosité. Ladite viscosité dynamique à 25°C est généralement comprise entre 3 et 35 Pa.s, mesurée avec un viscosimètre rotatif CONTRAVES TV équipé d'un mobile "MS-r4" à la fréquence de 60 Hz.
La composition sous forme de pâte souple peut être préparée, de manière connue, par mélange, chauffage et malaxage, ou bien par extrusion. L'utilisation d'un procédé par extrusion permet en particulier d'introduire plus de cires dans la composition qu'il ne serait possible d'en introduire par le procédé usuel.

L'extrusion permet également d'introduire dans la composition des cires, ou d'autres composés tels que des plastiques ou des polymères, ayant un point de fusion élevé. L'extrusion permet enfin d'introduire des composés volatils tout en évitant de porter la composition à une température trop élevée.

Les compositions selon l'invention trouvent une application notamment dans le domaine du maquillage de la peau et/ou des semi-muqueuses et/ou des muqueuses, et se présentent alors par exemple sous la forme d'un fond de teint, d'un fard à joues ou à paupières, d'un rouge à lèvres, d'un mascara ou d'un eye-liner.
Elles peuvent également être utilisées comme base de soin pour les lèvres ou comme base fixante à appliquer sur un rouge à lèvres classique. La base fixante forme alors un film protecteur sur le film de rouge, en limite le transfert et la migration, et permet d'augmenter ainsi sa tenue.
Elles peuvent encore se présenter sous la forme d'un produit de soin de la peau, des muqueuses et/ou des semi-muqueuses, d'un produit hygiénique ou pharmaceutique ou encore d'un produit solaire ou autobronzant.

Elles peuvent notamment comprendre des actifs cosmétiques et/ou pharmaceutiques. Parmi les agents actifs pharmaceutiquement, on peut citer les agents actifs contre les micro-organismes, notamment à activité antivirale, antibactérienne ou antifongique; les agents à activité anti-inflammatoire ou immunomodulatrice; les agents antagonistes des neuromédiateurs ou modulant le relarguage des neuromédiateurs; les agents modulant la différenciation et/ou la prolifération cellulaire et/ou la pigmentation et/ou régulant la kératinisation; les agents actifs dans le traitement et/ou la prévention des cheilites; les antihistaminiques; les agents cicatrisants.
Selon les pathologies à traiter, il peut être judicieux d'associer plusieurs agents actifs, ou encore de leur adjoindre des actifs secondaires qui vont favoriser la prévention ou le traitement de l'affection, et/ou traiter des symptômes associés à cette affection. Parmi les agents actifs secondaires, on peut citer les anesthésiques locaux, les antiseptiques, et les filtres solaires notamment chimiques ou minéraux. La composition peut en outre contenir un agent favorisant la solubilisation ou la compatibilité des agents actifs avec l'excipient de la composition, et/ou favorisant la pénétration desdits agents actifs dans la peau ou les muqueuses.

L'invention est illustrée plus en détails dans les exemples suivants.

### Exemple 1

On prépare un bâton de rouge à lèvres ayant la composition suivante:
. lanoline oxypropylénée (PPG5 lanolin wax; viscosité 19 Pa.s) 18 g
. triglycérides (hydrogenated cocoglycerides) 6 g
. cires hydrocarbonées (notamment polyéthylène) 17 g
. huile végétale 1,5 g
. pigments, nacres, charges 10 g
. cyclotétradiméthylsiloxane qsp 100 g

On prépare la composition de manière usuelle, en chauffant les corps gras, sauf les huiles volatiles, à 95°C et en les mélangeant. On ajoute ensuite les pigments et les charges, et, à 60°C, les huiles volatiles. On mélange le tout à l'aide d'une turbine Moritz à la vitesse de 3000 tr/min. On peut alors couler ledit mélange dans des moules adéquats.
On obtient ainsi un bâton de rouge à lèvres, de texture agréable, qui s'étale bien et s'applique uniformément. Le film est confortable à porter dans le temps et ne migre pas.
Il reste sur les lèvres, après évaporation du composé volatil, un film hydrocarboné de coloration homogène.

On applique cette composition sur la partie gauche des lèvres de plusieurs personnes.
Pour comparaison, on applique sur la partie droite desdites lèvres, la même composition (composition témoin) dans laquelle la lanoline et les triglycérides sont remplacés par des huiles végétales (huile de jojoba)
On laisse sécher les rouges à lèvres à température ambiante pendant 5 minutes, puis on applique la totalité des lèvres sur une feuille de papier.

On constate sur la totalité des feuilles de papier, une trace plus importante de rouge laissée par la composition témoin. La composition selon l'invention laisse sur la feuille une trace très faible, à peine perceptible.

### Exemple 2

On prépare un rouge à lèvres sous forme de pâte souple, ayant la composition suivante:
. lanoline acétylée (viscosité 20,5 Pa.s) 18 g
. triglycérides (hydrogenated cocoglycerides) 6 g
. huile végétale 1,5 g
. cires hydrocarbonées (notamment polyéthylène) 2,5 g
. pigments, nacres, charges 10 g
. cyclopentadiméthylsiloxane qsp 100 g

On introduit ces différents ingrédients, à l'exception de l'huile volatile, dans un extrudeur bi-vis, à une température d'environ 75-95°C en entrée.
On introduit l'huile volatile dans l'extrudeur en fin d'extrusion, à une température de l'ordre de 20-25°C.

On obtient en sortie une pâte souple se présentant sous forme d'une seule phase stable et homogène, et pouvant être prélevée à l'aide d'un pinceau pour son application.
Cette composition permet l'obtention d'un film homogène, facilement applicable et s'étalant facilement et uniformément. Le film obtenu présente également une texture légère et reste confortable à porter tout au long de la journée.
Il reste sur les lèvres, après évaporation du composé volatil, un film hydrocarboné de coloration homogène.

On applique cette composition sur la partie gauche des lèvres de plusieurs personnes.
Pour comparaison, on applique sur la partie droite desdites lèvres, la même composition sous forme de pâte souple (composition témoin) dans laquelle la lanoline et les triglycérides sont remplacés par des huiles végétales (huile de jojoba).
On laisse sécher les rouges à lèvres à température ambiante pendant 5 minutes, puis on applique la totalité des lèvres sur une feuille de papier.

On constate sur la totalité des feuilles de papier, une trace plus importante de rouge laissée par la composition témoin. La composition selon l'invention laisse sur la feuille une trace très faible, à peine perceptible.

### Exemple 3

On prépare une base fixante pour rouge à lèvres sous forme de bâton ayant la composition suivante:
. lanoline acétylée (viscosité 20,5 Pa.s) 18 g
. triglycérides 6 g
. huile végétale 1,5 g
. cires hydrocarbonées (notamment polyéthylène) 17 g
. charges 8 g
. isoparaffine qsp 100 g

Le film obtenu par application de la composition s'étale facilement et uniformément sur un rouge à lèvres classique.

## Revendications

1. Composition comprenant, dans une phase grasse, au moins un composé volatil, moins de 20% en poids d'huile non volatile hydrocarbonée, et 1-40% en poids d'au moins un composé gras pâteux présentant une viscosité à 40°C de 0,1 à 40 Pa.s et/ou un point de fusion de 25-70°C.

2. Composition selon la revendication 1, dans laquelle le composé gras pâteux présente une viscosité à 40°C de 0,5 à 25 Pa.s et/ou un point de fusion de 25-55°C.

3. Composition selon l'une des revendications précédentes dans laquelle le composé gras pâteux est un mélange de différents composés hydrocarbonés et/ou siliconés et/ou fluorés, les composés pâteux hydrocarbonés étant en proportion majoritaire.

4. Composition selon l'une des revendications précédentes dans laquelle le composé gras pâteux est un composé hydrocarboné ou un mélange de composé hydrocarbonés.

5. Composition selon l'une des revendications précédentes, dans laquelle le composé gras pâteux est choisi parmi, seul ou en mélange, les lanolines, les dérivés de lanoline; les esters gras, notamment ceux ayant 20 à 45 atomes de carbone; les triglycérides tels que les huiles végétales hydrogénées; le propionate d'arachidyle, le polylaurate de vinyle, les esters de cholestérol; les corps gras pâteux siliconés tels que les alkyls diméthicones; les huiles épaissies; les dérivés d'huile de ricin hydrogénée.

6. Composition selon la revendication 5, dans laquelle l'agent épaississant des huiles est choisi parmi les argiles telles que les bentonites ou les hectorites, éventuellement modifiées notamment par du chlorure de distéaryl diméthyl ammonium, ou par du chlorure de stéaryl diméthyl benzyl ammonium; les silicates d'aluminium ou de magnésium; les polymères susceptibles d'épaissir des huiles.

7. Composition selon l'une des revendications précédentes, dans laquelle le composé pâteux est présent à raison 8-35% en poids, de préférence 15-30% en poids.

8. Composition selon l'une des revendications précédentes, dans laquelle le composé volatil est choisi parmi les huiles hydrocarbonées ou les huiles siliconées, cycliques ou linéaires, seules ou en mélange.

9. Composition selon l'une des revendications précédentes, comprenant 8-70% en poids, de préférence 15-55% en poids, de composés volatils par rapport au poids total de la composition.

10. Composition selon l'une des revendications précédentes, comprenant moins de 15% en poids d'huile non volatile hydrocarbonée, de préférence moins de 5% en poids, voire pas d'huiles hydrocarbonées non volatiles du tout.

11. Composition selon l'une des revendications précédentes, dans laquelle la phase grasse comprend en outre au moins une cire.

12. Composition selon la revendication 11, dans laquelle la cire est choisie parmi, seules ou en mélange, les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; les cires de silicone.

13. Composition selon l'une des revendications 11 à 12, comprenant 10-30% en poids de cire, de préférence 15-20% en poids.

14. Composition selon l'une des revendications précédentes, dans laquelle la phase grasse comprend uniquement des corps gras hydrocarbonés.

15. Composition selon la revendication 14, dans laquelle la phase grasse comprend des composés gras pâteux de type lanoline, des composés volatils de type isoparaffines et des cires hydrocarbonées.

16. Composition selon l'une des revendications précédentes, se présentant sous la forme d'un stick ou bâton, sous la forme d'un liquide huileux, éventuellement gélifié, ou encore sous la forme d'une pâte souple ayant une viscosité dynamique comprise entre 3 et 35 Pa.s.

17. Composition selon l'une des revendications précédentes, se présentant sous forme d'un produit hygiénique, pharmaceutique, de soin ou de maquillage de la peau, des semi-muqueuses et/ou des muqueuses, tel qu'un fond de teint, un fard à joues ou à paupières, un rouge à lèvres, un mascara, un eye-liner, une base de soin pour les lèvres, une base fixante à appliquer sur un rouge à lèvres, un produit solaire ou autobronzant.

18. Composition sans transfert de maquillage de la peau et/ou des lèvres, comprenant 1-40% en poids d'au moins un composé gras pâteux présentant une viscosité à 40°C de 0,1 à 40 Pa.s et/ou un point de fusion de 25-70°C, au moins un composé volatil et moins de 20% en poids d'huile non volatile hydrocarbonée.

19. Utilisation de l'association d'au moins un composé volatil et d'au moins un composé gras pâteux présentant une viscosité à 40°C de 0,1 à 40 Pa.s et/ou un point de fusion de 25-70°C, afin de diminuer le transfert et/ou la migration, et/ou afin d'améliorer la tenue d'une composition les comprenant.

20. Utilisation selon la revendication 19, dans une composition comprenant moins de 20% en poids d'huile non volatile hydrocarbonée, notamment moins de 15% en poids d'huile non volatile hydrocarbonée, de préférence moins de 5% en poids, voire pas d'huiles hydrocarbonées non volatiles du tout.

21. Utilisation selon l'une des revendications 19-20, dans laquelle le composé gras pâteux présente une viscosité à 40°C de 0,5 à 25 Pa.s et/ou un point de fusion de 25-55°C.

22. Utilisation selon l'une des revendications 19-21, dans laquelle le composé gras pâteux est choisi parmi, seul ou en mélange, les lanolines, les dérivés de lanoline; les esters gras, notamment ceux ayant 20 à 45 atomes de carbone; les triglycérides tels que les huiles végétales hydrogénées; le propionate d'arachidyle, le polylaurate de vinyle, les esters de cholestérol; les corps gras pâteux siliconés tels que les alkyls diméthicones; les huiles épaissies; les dérivés d'huile de ricin hydrogénée.

23. Utilisation selon l'une des revendications 19-22, dans laquelle le composé pâteux est présent dans la composition à raison de 1 à 40% en poids, de préférence à raison de 8-35% en poids et encore plus préférentiellement à raison de 15-30% en poids.

24. Utilisation selon l'une des revendications 19-23, dans une composition dont la phase grasse comprend uniquement des corps gras hydrocarbonés, notamment des composés gras pâteux de type lanoline, des composés volatils de type isoparaffines et des cires hydrocarbonées.

25. Utilisation selon l'une des revendications 19-24, dans une composition se présentant sous forme d'un produit hygiénique, pharmaceutique, de soin ou de maquillage de la peau, des semi-muqueuses et/ou des muqueuses, tel qu'un fond de teint, un fard à joues ou à paupières, un rouge à lèvres, un mascara, un eye-liner, une base de soin pour les lèvres, une base fixante à appliquer sur un rouge à lèvres, un produit solaire ou autobronzant.

26. Procédé pour limiter, diminuer et/ou empêcher le transfert d'une composition comprenant des composés volatils, notamment une composition de maquillage ou de soin de la peau, des muqueuses, des semi-muqueuses et/ou des phanères, consistant à introduire dans ladite composition au moins un composé gras pâteux.
